# EUROPÄISCHE PATENTSCHRIFT

(11) **EP 1 711 156 B1**
(45) Veröffentlichungstag und Bekanntmachung des Hinweises auf die Patenterteilung: **06.05.2009**
(21) Anmeldenummer: 04790615.1
(22) Anmeldetag: 19.10.2004
(51) Int. Cl.: A61K 8/18

(54) **PERLGLANZENDES FARBEMITTEL FUR KERATINFASERN**
NACREOUS LUSTER-EFFECT DYE FOR KERATIN FIBERS
MATIERE COLORANTE A LUSTRE NACRE POUR FIBRES KERATINIQUES

(30) Priorität: 05.02.2004 DE 102004005768
(43) Veröffentlichungstag der Anmeldung: 18.10.2006
(73) Patentinhaber: The Procter and Gamble Company, Cincinnati, Ohio 45202 (US)
(72) Erfinder: SCHMENGER, Jürgen, 64331 Weiterstadt (DE); KUJAWA, Jolanthe, 64289 Darmstadt (DE)
(74) Vertreter: Bockhorst, Matthias
(86) Internationale Anmeldenummer: PCT/EP2004/011790
(87) Internationale Veröffentlichungsnummer: WO 2005/074870

(56) Entgegenhaltungen:
- DE-A1- 3 834 142
- DE-A1- 10 240 276
- DE-C1- 19 701 422
- US-A- 4 555 246
- US-A1- 2002 046 431
- US-A1- 2003 135 936
- US-B1- 6 528 045
- US-B1- 6 562 772

## Beschreibung

Gegenstand der Erfindung sind perlmuttartig glänzende Mittel zum Färben von Keratinfasern, insbesondere menschlichen Haaren, mit einem Gehalt an direktziehenden und/oder oxidativen Farbstoffen und einer speziellen Kombination aus Fettalkoholen, Alkanolamiden, Alkoxylaten und anionischen Tensiden, sowie die Verwendung der vorgenannten Kombination zur Erzeugung eines stabilen Perlglanzes in Haarfärbemitteln.

Färbende Präparate liegen üblicherweise in Form von wässrigen -vorzugsweise verdickten- Lösungen oder Emulsionen vor und enthalten neben Farbstoffen beispielsweise Fettalkohole und/oder andere Ölkomponenten, Emulgatoren und Tenside, sowie gegebenenfalls Alkohole. Oxidationsfärbemittel bestehen in der Regel aus zwei Komponenten, (i) der die Farbstoffe enthaltenden Farbstoffträgermasse und (ii) der Oxidationsmittelzubereitung, die kurz vor dem Gebrauch miteinander vermischt und dann auf das zu färbende Haar aufgetragen werden. Liegen die färbenden Präparate als Emulsionen vor, so sind diese in der Regel stabile Cremes, die jedoch für die Erzeugung eines Perlmuttglanzeffektes den Zusatz von speziellen Perlglanzmitteln benötigen.

Aus der DE-A 38 34 142 sind cremeförmige Haarfärbemittel bekannt, welche eine Vielzahl von Rohstoffen, u.a. auch Fettalkohole und Fettsäurealkanolamide sowie anionische und nichtionische Tenside enthalten. Ebenfalls sind aus der DE-C 197 01 422 Haarfärbemittel bekannt, welche eine Vielzahl von Rohstoffen, u.a. auch Fettalkohole und Fettsäurealkanolamide sowie anionische und nichtionische Tenside enthalten. Die aus dem Stand der Technik bekannten Mittel weisen jedoch keinen Perlglanz auf.

Es bestand daher die Aufgabe, eine Farbmasse zu entwickeln, die ohne den Zusatz von Perlglanzmitteln, alleine durch die Auswahl der Rohstoffe einen stabilen perlmuttartigen Charakter aufweist, der auch nach dem Vermischen mit der Oxidationsmittelzubereitung bestehen bleibt. Weiterhin soll der Pflegeeffekt nach dem Ausspülen der Farbmasse gegenüber Formulierungen nach dem Stand der Technik verbessert werden.

Es wurde nunmehr gefunden, dass durch Verwendung einer Kombination aus einem Fettalkohol, einem Alkanolamid, einem Alkoxylat und einem anionischen Tensid diese Aufgabe in hervorragender Weise gelöst wird.

Gegenstand der vorliegenden Erfindung ist daher eine Farbträgermasse, enthaltend oxidative und/oder nicht-oxidative ("direktziehende") Farbstoffe, dadurch gekennzeichnet, dass sie eine Kombination aus
(a) 6,1 bis 20 Gewichtsprozent mindestens eines Fettalkoholes mit 14 bis 20 Kohlenstoffatomen,
(b) 6,1 bis 20 Gewichtsprozent mindestens eines Alkanolamides,
(c) 0,1 bis 15 Gewichtsprozent mindestens eines Fettalkoholalkoxylates oder Fettsäurealkoxylates und
(d) 0,1 bis 15 Gewichtsprozent mindestens eines anionischen Tensides enthält,
wobei das Gewichtsverhältnis von Fettalkohol (a) zu Alkanolamid (b) gleich 0,5:2 bis 2:0,5 ist.

Besonders bevorzugt sind Farbträgermassen, bei denen der Fettalkohol (a) und das Alkanolamid (b) in einem Gewichtsverhältnis von 0,8:1,2 bis 1,2:0,8 vorliegen.

Für die Bildung eines besonders schönen Perlmuttcharakters sowie eines besonders hohen Pflegeeffektes nach dem Ausspülen der Farbmasse ist es zudem von Vorteil, wenn das Gewichtsverhältnis von Alkoxylat (c) zu anionischem Tensid (d) gleich 0,5:2 bis 2:0,5 ist, wobei ein Gewichtsverhältnis von (c) zu (d) von 0,8:1,2 bis 1,2:0,8 besonders bevorzugt ist.

Erfindungsgemäß geeignete langkettige Fettalkohole mit 14 bis 20 Kohlenstoffatomen sind zum Beispiel Cetylakohol, Stearylalkohol, Myristylalkohol, Isooctylalkohol oder Isotridecylalkohol. Die Fettalkohole können in der erfindungsgemäßen Farbträgermasse sowohl einzeln als auch in Kombination miteinander eingesetzt werden.

Erfindungsgemäß geeignete Alkanolamide sind insbesondere die N-Acylderivate des Monoethanolamins oder Diethanolamins, beispielsweise Monoethanolamide und Diethanolamide, oder Esteramide wie das Kokosnußfettsäuremonoethanolamid.

Als erfindungsgemäß geeignetes Alkoxylat (c) können insbesondere ethoxylierte Fettalkohole oder Fettalkoholpolyglykoether der nachstehenden Formel (I) genannt werden:

CH3(CH2)x-O-(Ry)-H (I)

[mit R= (CH2-CH2-O) oder (CH3-CH-CH2-O); x= C8 - C18 und y= 2 bis 300]

Besonders bevorzugte Fettalkoholalkoxylate sind beispielsweise Polyethylenglykolether des Stearylalkohols wie zum Beispiel Steareth-10, Ceteareth-25 oder Steareth-20.

Erfindungsgemäß geeignete anionische Tenside sind Salze und Ester von Carbonsäuren, Alkylethersulfate und Alkylsulfate, Fettalkoholethersulfate, Sulfonsäure und ihre Salze (Sulfosuccinate, Fettsäureisethinate, usw.), Phosphorsäureester und ihre Salze, Acylaminosäuren und ihre Salze.

Eine ausführliche Beschreibung dieser anionischen Tenside ist der Publikation "FIEDLER - Lexikon der Hilfsstoffe", Band 1, fünfte Auflage (2002), Seiten 97 bis 102, zu entnehmen, auf die hiermit ausdrücklich Bezug genommen wird.

Der Fettalkohol der Komponente (a) sowie das Alkanolamid der Komponente (b) werden in der erfindungsgemäßen Farbträgermasse vorzugsweise jeweils in einer Gesamtmenge von 7 bis 12 Gewichtsprozent, eingesetzt.

Das Alkoxylat der Komponente (c) sowie das anionische Tensid der Komponente (d) werden in der erfindungsgemäßen Farbträgermasse vorzugsweise jeweils in einer Gesamtmenge von 7 bis 12 Gewichtsprozent, eingesetzt.

Vorzugsweise ist die erfindungsgemäße Farbträgermasse frei von monomeren quaternären Ammoniumverbindungen sowie kationischen Emulgatoren und Tensiden und enthält gegebenenfalls 0,1 bis 10 Gewichtsprozent, bevorzugt 0,5 bis 4 Gewichtsprozent, Glykoldistearat.

Die erfindungsgemäße Farbträgermasse enthält vorzugsweise Oxidationsfarbstoffvorstufen, bei denen die Färbung unter Einwirkung von Oxidationsmitteln, wie zum Beispiel Wasserstoffperoxid und dessen Addukten, oder in Gegenwart von Luftsauerstoff erzeugt wird.

Als geeignete Oxidationsfarbstoffvorstufen können beispielsweise die folgenden Entwicklersubstanzen und Kupplersubstanzen und mit sich selbst kuppelnden Verbindungen genannt werden:
(i) Entwicklersubstanzen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluorphenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1 H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1 H-pyrazol, 4,5-Diamino-1-methyl-1 H-pyrazol, 2-Amino-phenol, 2-Amino-6-methyl-phenol, 2-Amino-5-methyl-phenol, allein oder im Gemisch miteinander.
(ii) Kupplersubstanzen: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxybenzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Aminophenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, allein oder im Gemisch miteinander.
(iii) Mit sich selbst kuppelnde Verbindungen: 2-Amino-5-methylphenol, 2-Amino-6-methylphenol, 2-Amino-5-ethoxyphenol oder 2-Propylamino-5-aminopyridin.

Die Gesamtmenge der in der erfindungsgemäßen Farbträgermasse enthaltenen Oxidationsfarbstoffvorstufen beträgt etwa 0,01 bis 12 Gewichtsprozent, insbesondere etwa 0,2 bis 6 Gewichtsprozent.

Zur Erzielung bestimmter Farbnuancen können ferner auch übliche natürliche und/oder synthetische direktziehende Farbstoffe, beispielsweise sogenannte Pflanzenfarbstoffe wie Henna oder Indigo,Triphenylmethanfarbstoffe, aromatische Nitrofarbstoffe, Azofarbstofte, Chinonfarbstoffe, kationische oder anionische Farbstoffe, in dem Färbemittel enthalten sein.

Als geeignete synthetische Farbstoffe können beispielsweise genannt werden: 1,4-Bis[(2-hydroxyethyl)amino]-2-nitrobenzol, 1-(2-Hydroxyethyl)-amino-2-nitro-4-[di(2-hydroxyethyl)-amino]-benzol (HC Blue No. 2), 1-Amino-3-methyl-4-[(2-hydroxyethyl)-amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxy-ethyl)-amino]-2-nitrobenzol (HC Blue No. 10), 1-[(2,3-Dihydroxypropyl)-amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxy-propyl)amino]-2-nitrobenzol (HC Blue No. 6), 2-((4-Amino-2-nitrophenyl)-amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitrodiphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxy-propoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)-amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxy-ethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-[(2-Hydroxy-ethyl)-amino]-1-methoxy-5-nitrobenzol, 2-Amino-3-nitrophenol, 1-(2-Hydroxyethoxy)-3-methylamino-4-nitrobenzol, 2,3-(Dihydroxy-propoxy)-3-methyl-amino-4-nitrobenzol, 2-[(2-Hydroxyethyl)amino]-5-nitrophenol (HC Yellow No. 11), 3-[(2-Aminoethyl)-amino]-1-methoxy-4-nitrobenzol-hydrochlorid (HC Yellow No.9), 1-[(2-Ureidoethyl)amino]-4-nitrobenzol, 4-[(2,3-Di-hydroxypropyl)amino]-3-nitro-1-trifluormethyl-benzol (HC Yellow No. 6), 1-Chlor-2,4-bis[(2-hydroxyethyl)-amino]-5-nitrobenzol (HC Yellow No. 10), 4-[(2-Hydroxy-ethyl)amino]-3-nitro-1-methylbenzol, 1-Chlor-4-[(2-hydroxy-ethyl)amino]-3-nitrobenzol (HC Yellow No. 12), 4-[(2-Hydroxyethyl)-amino]-3-nitro-1-trifluormethyl-benzol (HC Yellow No. 13), 4-[(2-Hydroxy-ethyl)amino]-3-nitro-benzonitril (HC Yellow No. 14), 4-[(2-Hydroxyethyl)-amino]-3-nitro-benzamid (HC Yellow No. 15), 1,4-Di[(2,3-dihydroxy-propyl)amino]-9,10-anthrachinon, 1-[(2-Hydroxy-ethyl)amino]-4-methyl-amino-9,10-anthrachinon (CI61505, Disperse Blue No. 3), 2-[(2-Amino-ethyl)amino]-9,10-anthrachinon (HC Orange No. 5), 1-Hydroxy-4-[(4-methyl-2-sulfophenyl)amino]-9,10-anthrachinon, 1-[(3-Aminopropyl)-amino]-4-methylamino-9,10-anthrachinon (HC Blue No. 8),1-[(3-Amino-propyl)-amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 9-(Dimethylamino)-benzo[a]-phenoxazin-7-ium-chlorid (CI51175; Basic Blue No. 6), Di[4-(diethyl-amino)phenyl][4-(ethylamino)naphthyl]-carbenium-chlorid (CI42595; Basic Blue No. 7), 3,7-Di(dimethylamino)-phenothiazin-5-iumchlorid (CI52015; Basic Blue No. 9), Di[4-(dimethyl-amino)phenyl][4-(phenylamino)naphthyl]-carbenium-chlorid (CI44045; Basic Blue No. 26), 2-[(4-(Ethyl(2-hydroxy-ethyl)amino)phenyl)azo]-6-methoxy-3-methylbenzothiazolium-methylsulfat (CI11154; Basic Blue No. 41), 8-Amino-2-brom-5-hydroxy-4-imino-6-[(3-(trimethylammonio)phenyl)-amino]-1(4H)-naphthalinon-chlorid (CI56059; Basic Blue No. 99), Bis[4-(dimethylamino)phenyl][4-(methyl-amino)phenyl]carbenium-chlorid (CI42535; Basic Violet No. 1), Tris[4-(dimethylamino)phenyl]carbenium-chlorid (CI42555; Basic Violet No. 3), 2-[3,6-(Diethylamino)-dibenzopyranium-9-yl]-benzoesäure-chlorid (CI45170; Basic Violet No. 10), Di(4-aminophenyl)-(4-amino-3-methyl-phenyl)carbenium-chlorid (CI42510; Basic Violet No. 14), 1,3-Bis[(2,4-diamino-5-methylphenyl)azo]-3-methylbenzol (CI21010; Basic Brown No. 4), 1-[(4-Aminophenyl)azo]-7-(trimethylammonio)-2-naphthol-chlorid (CI12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethyl-ammonio)-2-naphtholchlorid (CI12251; Basic Brown No. 17), 3,7-Diamino-2,8-dimethyl-5-phenyl-phenazinium-chlorid (CI50240; Basic Red No. 2), 1,4-Dimethyl-5-[(4-(dimethylamino)phenyl)-azo]-1,2,4-triazolium-chlorid (CI11055; Basic Red No. 22), 2-Hydroxy-1-[(2-methoxy-phenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)-phenyl)azo]-pyrazol-5-on-chlorid (CI12719; Basic Yellow No. 57), Bis[4-(diethylamino)phenyl]phenylcarbenium-hydrogensulfat(1:1) (CI42040; Basic Green No. 1), 1-[Di(2-hydroxyethyl)-amino]-3-methyl-4-[(4-nitro-phenyl)azo]-benzol (CI11210, Disperse Red No. 17), 4-[(4-Aminophenyl)-azo]-1-[di(2-hydroxyethyl)amino]-3-methyl-benzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Mono- und Disulfonsäure) (CI47005;D&C Yellow No. 10; Food Yellow No. 13; Acid Yellow No. 3), 5-Hydroxy-1-(4-sulfophenyl)-4-[(4-sulfophenyl)-azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 5-[(2,4-Dinitrophenyl)-amino]-2-phenylamino-benzolsulfonsäure-natriumsalz (CI10385; Acid Orange No. 3), 4-[(2,4-Dihydroxyphenyl)azo]-benzolsulfonsäuremononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäure-dinatriumsalz (CI14720; Acid Red No. 14), 6-Hydroxy-5-[(4-sulfonaphth-1-yl)azo]-2, 4-naphthalindisulfonsäure-trinatriumsalz (CI16255; Ponceau 4R; Acid Red No. 18), 3-Hydroxy-4-[(4-sulfonaphth-1-yl)azo]-2,7-naphthalin-disulfonsäuretrinatriumsalz (CI16185; Acid Red No. 27), 8-Amino-1-hydroxy-2-(phenylazo)-3,6-naphthalin-disulfonsäure-dinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäuredinatriumsalz (CI45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)-phenyl)azo]-7-naphthol-1,3-disulfonsäure-dinatriumsalz (CI27290; Acid Red No. 73), 2',4',5',7'-Tetrabrom-3',6'-dihydroxyspiro-[isobenzofuran-1(3H),9'-[9H]xanthen]-3-on-dinatriumsalz (CI45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro[isobenzofuran-1(3H),9'(9H)-xanthen]-3-on-dinatriumsalz (CI45425; Acid Red No. 95), (2-Sulfophenyl)di[4-(ethyl((4-sulfophenyl)methyl)amino)phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxynaphth-1-yl)carbenium-inneres Salz, mononatriumsalz (CI44090; Food Green No. 4; Acid Green No. 50), Bis[4-(diethylamino)phenyl](2,4-disulfophenyl)carbenium-inneres salz, Natriumsalz (2:1) (CI42045; Food Blue No. 3; Acid Blue No. 1), Bis[4-(diethylamino)phenyl](5-hydroxy-2,4-disulfophenyl)carbenium-inneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62),2-(1,3-Dihydro-3-oxo-5-sulfo-2H-indol-2-yliden)-2,3-dihydro-3-oxo-1H-indol-5-sulfonsäuredinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalindisulfonsäure-dinatriumsalz (CI20470; Acid Black No. 1), 3-Hydroxy-4-[(2-hydroxynaphth-1-yl)azo]-7-nitro-1-naphthalin-sulfonsäurechromkomplex (3:2) (CI15711; Acid Black No. 52), 3-[(2,4-Dimethyl-5-sulfophenyl)azo]-4-hydroxy-1-naphthalin-sulfonsäure-dinatriumsalz (CI14700; Food Red No. 1; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1) und 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red No. 195), alleine oder in Kombination miteinander.

Die Gesamtmenge der direktziehenden Farbstoffe beträgt in der erfindungsgemäßen Farbträgermasse etwa 0,01 bis 7 Gewichtsprozent, vorzugsweise etwa 0,2 bis 4 Gewichtsprozent.

Weitere zur Haarfärbung bekannte und übliche Farbstoffe, die in dem erfindungsgemäßen Färbemittel enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782 bis 815 beschrieben, auf die hiermit ausdrücklich Bezug genommen wird.

Obwohl Oxidationsfärbemittel bevorzugt sind, ist es selbstverständlich ebenfalls möglich, dass die erfindungsgemäße Farbträgermasse in Form eines nicht-oxidativen Färbemittels auf Basis der vorstehend genannten direktziehenden Farbstoffen vorliegt.

Darüberhinaus können in der erfindungsgemäßen Farbträgermasse Antioxidantien wie zum Beispiel Ascorbinsäure, Thioglykolsäure oder Natriumsulfit, sowie Komplexbildner für Schwermetalle, beispielsweise Ethylendiaminotetraacetat oder Nitriloessigsäure, in einer Menge von bis zu etwa 0,5 Gewichtsprozent enthalten sein. Parfümöle können in der erfindungsgemäßen Farbträgermasse in einer Menge von bis zu etwa 1 Gewichtsprozent enthalten sein. Selbstverständlich kann die vorstehend beschriebene Farbträgermasse gegebenenfalls weitere, für Haarfärbemittel übliche Zusätze, wie zum Beispiel Verdickungsmittel, beispielsweise Homopolymere der Acrylsäure, Pflanzen Gums, Cellulose- und Stärkederivate, Algenpolyasaccharide, amphiphile Assoziatiwerdicker, desweiteren Konservierungsstoffe; Antioxidantien, beispielsweise Natriumsulfit, Thioglykolsäure oder Ascorbinsäure; Komplexbildner; Lösungsmittel wie Wasser, niedere aliphatische Alkohole, beispielsweise aliphatische Alkohole mit 1 bis 4 Kohlenstoffatomen wie Ethanol, Propanol und Isopropanol, oder Glykole wie Glycerin und 1,2-Propylenglykol; Netzmittel oder Emulgatoren die nicht in den Hauptansprüchen genannt sind, aus den Klassen der anionischen, amphoteren oder nichtionogenen oberflächenaktiven Substanzen; weiterhin Weichmacher; Vaseline; Silikonöle, Paraffinöl, Polysorbate und Fettsäuren sowie außerdem Pflegestoffe, wie kationische Polymere oder Harze, Lanolinderivate, Cholesterin, Vitamine, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,1 bis 30 Gewichtsprozent und die Pflegestoffe in einer Konzentration von 0,1 bis 5,0 Gewichtsprozent.

Der pH-Wert der erfindungsgemäßen Farbträgermasse liegt bei nicht-oxidativen Färbemitteln auf der Basis von direktziehenden Farbstoffen im Bereich von etwa 5 bis 10, vorzugsweise 6 bis 9, während bei oxidativen Färbemitteln auf der Basis von Oxidationsfarbstoffvorstufen der pH-Wert in einem Bereich von etwa 6 bis 12, vorzugsweise 9 bis 11, liegt, wobei der pH-Wert des gebrauchsfertigen Oxidationshaarfärbemittels (das heißt der Mischung der erfindungsgemäßen Farbträgermasse mit dem Oxidationsmittel) etwa 5,5 bis 10, vorzugsweise 6 bis 9, beträgt.

Je nach Zusammensetzung und gewünschtem pH-Wert erfolgt die Einstellung des pH-Wertes vorzugsweise mit Ammoniak oder organischen Aminen, wie zum Beispiel Glucaminen, Aminomethyl-propanol, Monoethanolamin oder Triethanolamin, anorganischen Basen, beispielsweise Natriumhydroxid, Kaliumhydroxid, Natriumcarbonat oder Calciumhydroxid, beziehungsweise organischen oder anorganischen Säuren, wie zum Beispiel Milchsäure, Zitronensäure, Essigsäure oder Phosphorsäure.

Die erfindungsgemäße Farbträgermasse wird vorzugsweise in Form einer wässrigen oder wässrig-alkoholischen Zubereitung, beispielsweise als verdickte Lösung, als Emulsion, als Creme oder als Gel, konfektioniert.

Für die Anwendung zur oxidativen Färbung vermischt man die vorstehend beschriebene Farbträgermasse unmittelbar vor dem Gebrauch mit einem Oxidationsmittel und trägt eine für die Färbung ausreichende Menge, in der Regel etwa 60 bis 200 Gramm, der gebrauchsfertigen Zubereitung auf die Faser auf.

Sofern die erfindungsgemäße Farbträgermasse keine Oxidationsfarbstoffvorstufen enthält beziehungsweise Oxidationsfarbstoffvorstufen enthält, welche mit Luftsauerstoff leicht oxidierbar sind, kann sie ohne vorheriges Vermischen mit einem Oxidationsmittel direkt auf die Keratinfaser aufgetragen werden.

Als Oxidationsmittel zur Entwicklung der Färbung kommen hauptsächlich Wasserstoffperoxid oder dessen Additionsverbindungen an Harnstoff, Melamin oder Natriumborat in Form einer 1- bis 12prozentigen, vorzugsweise 1,5- bis 6prozentigen wässrigen Lösung in Betracht. Das Mischungsverhältnis von Farbträgermasse zu Oxidationsmittel ist abhängig von der Konzentration des Oxidationsmittels und beträgt in der Regel etwa 5:1 bis 1:2, vorzugsweise 1:1, wobei der Gehalt an Oxidationsmittel in der gebrauchsfertigen Zubereitung vorzugsweise etwa 0,5 bis 8 Gewichtsprozent, insbesondere 1 bis 4 Gewichtsprozent, beträgt.

Man läßt das gebrauchsfertige Färbemittel bei 15 bis 50 °C etwa 10 bis 45 Minuten, vorzugsweise etwa 15 bis 30 Minuten lang, auf die Keratinfaser (zum Beispiel menschliche Haare) einwirken, spült sodann die Faser mit Wasser aus. Gegebenenfalls wird im Anschluß an diese Spülung mit einem Shampoo gewaschen und eventuell mit einer schwachen organischen Säure, wie zum Beispiel Weinsäure, nachgespült. Abschließend wird die Keratinfaser getrocknet.

Die erfindungsgemäße Farbträgermasse weist eine gleichmäßige Konsistenz sowie eine sehr kosmetische perlmuttglänzende Anmutung auf. Ein mit der erfindungsgemäßen Farbträgermasse hergestelltes Färbemittel erfüllt die in Bezug auf die Hafteigenschaften, das Auftrageverhalten und die Viskositätseinstellung gestellten Anforderungen in hervorragenderer Weise und bietet durch den perlmuttartigen Charakter ein überaus kosmetisches Aussehen. Zudem wird ein gegenüber bekannten Färbemitteln deutlich verbessertes Pflegeergebnis nach dem Ausspülen erzielt.

Ein weiterer Gegenstand der vorliegenden Anmeldung ist die Verwendung einer Kombination aus
(a) mindestens einem Fettalkohol mit 14 bis 20 Kohlenstoffatomen,
(b) mindestens einem Alkanolamid,
(c) mindestens einem Fettalkoholalkoxylat oder Fettsäurealkoxylat und
(d) mindestens einem anionischen Tensid, wobei das Gewichtsverhältnis von Fettalkohol (a) zu Alkanolamid (b) gleich 0,5:2 bis 2:0,5 ist,
zur Erzeugung eines Perlglanzeffektes in Farbträgermassen und Färbemitteln für Keratinfasern, insbesondere menschlichen Haaren.

Besonders bevorzugt ist die Verwendung einer Kombination aus
(a) 6,1 bis 20 Gewichtsprozent, insbesondere 7 bis 12 Gewichtsprozent, mindestens eines Fettalkoholes mit 14 bis 20 Kohlenstoffatomen,
(b) 6,1 bis 20 Gewichtsprozent, insbesondere 7 bis 12 Gewichtsprozent, mindestens eines Alkanolamides,
(c) 0,1 bis 15 Gewichtsprozent, insbesondere 7 bis 12 Gewichtsprozent, mindestens eines Fettalkoholalkoxylates oder Fettsäurealkoxylates und
(d) 0,1 bis 15 Gewichtsprozent, insbesondere 7 bis 12 Gewichtsprozent, mindestens eines anionischen Tensides, wobei das Gewichtsverhältnis von Fettalkohol (a) zu Alkanolamid (b) gleich 0,5:2 bis 2:0,5, insbesondere 0,8:1,2 bis 1,2:0,8, ist.

Die nachfolgenden Beispiele sollen den Gegenstand der Erfindung näher erläutern ohne diesen hierauf zu beschränken.

### Beispiele

| **Beispiel 1:** | **Oxidationshaarfärbemittel, cremförmig** |
|---|---|
| 6,0000 g | Stearylalkohol |
| 5,0000 g | Cetylalkohol |
| 8,0000 g | Kokosfettsäuremonoethanolamid (Cocamide MEA) |
| 4,0000 g | Polyoxyerthylen(10)stearylether (Steareth-10) |
| 8,0000 g | Natriumlaurylalkoholdiglykolethersulfat, 28%ige wässrige Lösung |
| 1,3620 g | 4-Aminophenol |
| 0,5000 g | 1-Naphthol |
| 0,0136 g | Resorcin |
| 0,0034 g | 2-Amino-6-chlor-4-nitrophenol |
| 12,0000 g | Ammoniak, 25%ige wässrige Lösung |
| 1,0000 g | Ethylendiaminoteraacetat-Dinatriumsalz |
| 1,0000 g | Ascorbinsäure |
| ad 100,0000 g | Wasser |

50 g der vorstehenden perlmuttglänzenden Farbträgermasse werden unmittelbar vor Gebrauch mit 50 g einer 6prozentigen wässrigen Wasserstoffperoxidlösung vermischt. Es wird eine homogene, perlmuttgänzende, kosmetisch anmutende Färbezubereitung erhalten.

Das so erhaltene Gemisch wird anschließend auf blonde Naturhaare aufgetragen. Nach einer Einwirkungszeit von 30 Minuten bei 40 °C wird das Haar mit Wasser gespült und getrocknet. Das Haar erhält eine leuchtende, kupferrote Färbung.

| **Beispiel 2:** | **Oxidationshaarfärbemittel zur Hellerfärbung** |
|---|---|
| Komponente (A): | Cremige Farbträgermasse |
| 12,00 g | Cetystearyllalkohol |
| 8,00 g | Kokosfettsäuremonoethanolamid (Cocamide MEA) |
| 6,00 g | Polyoxyethylen(25)cetylstearylether (Ceteareth-25) |
| 1,00 g | Ölsäure |
| 4,00 g | Natriumlaurylalkoholdiglykolethersulfat, 70%ige wässrige Lösung |
| 0,50 g | p-Phenylendiamin |
| 0,07 g | Resorcin |
| 1,00 g | Ethylendiamintetraessigsäure-Dinatriumsalz |
| 8,00 g | Ammoniak, 25%ige wässrige Lösung |
| 8,00 g | Ethanol |
| ad 100,00 g | Wasser |

| Komponente (B): | Wasserstoffperoxid-Emulsion |
|---|---|
| 10,0 g | Cetylstearylalkohol |
| 1,5 g | Cholesterin |
| 4,0 g | Natriumlaurylalkoholdiglykolethersulfat, 28%ige wässrige Lösung |
| 35,0 g | Wasserstoffperoxid,35 %ige wässrige Lösung |
| 0,3 g | Parfüm |
| ad 100,0 g | Wasser |

Man vermischt vor dem Gebrauch 40 g der flüssigen perlglänzenden Farbträgermasse (A) mit 80 g der Wasserstoffperoxid-Emulsion (B), entsprechend einem Mischungsverhältnis von (A):(B) von 1:2, und trägt 120 g dieses Gemisches auf graues, menschliches Haar auf. Nach einer Einwirkungszeit von 20 Minuten bei Raumtemperatur wird das Haar mit Wasser ausgespült getrocknet. Das so behandelte Haar ist vom Haaransatz bis zu den Haarspitzen gleichmäßig hellbraun gefärbt. Das genrauchsfertige Mittel weist einen kosmetischen Perlglanz auf und ist leicht auf das Haar auftragbar und läuft nicht vom Haar ab.

| **Beispiel 3:** | **Oxidationshaarfärbemittel, cremeförmig** |
|---|---|
| 4,00 g | Cetylstearylakohol |
| 5,00 g | Stearylalkohol |
| 12,00 g | Kokosfettsäuremonoethanolamid (Cocamide MEA) |
| 2,00 g | Polyoxyethylen(20)stearylether (Steareth-20) |
| 8,00 g | Natriumlaurylalkoholdiglykolethersulfat, 28%ige wässrige Lösung |
| 8,00 g | Monoethanolamin |
| 1,30 g | 1-Methyl-2,5-diaminobenzol |
| 1,50 g | Diallyldiammoniumchlorid/Hydroxyethylcellulose-Copolymer (Polyquaternium-4) |
| 0,65 g | Resorcin |
| 0,50 g | Keratinhydrolysat |
| 0,50 g | Seidenproteinhydrolysat |
| 0,52 g | 2-Amino-6-chlor-4-nitrophenol |
| 1,00 g | Ethylendiaminotetraacetat-Dinatriumsalz |
| 0,30 g | Ascorbinsäure |
| ad 100,00 g | Wasser |

50 g der vorstehenden perlglänzenden Farbträgermasse werden unmittelbar vor Gebrauch mit 50 g einer 12prozentigen wässrigen Wasserstoffperoxidlösung vermischt. Das erhaltene Gemisch wird anschließend auf blonde Naturhaare aufgetragen. Nach einer Einwirkungszeit von 30 Minuten bei 40 °C wird das Haar mit Wasser gespült und getrocknet. Es wird ein gleichmäßiger, kräftiger Braunton erhalten.

| **Beispiel 4:** | **nicht-oxidatives Haarfärbemittel** |
|---|---|
| 6,000 g | Stearylalkohol |
| 4,000 g | Myristylalkohol |
| 8,000 g | Kokosfettsäuremonoethanolamid (Cocamide MEA) |
| 2,000 g | Polyoxyethylen(20)stearylether (Steareth-20) |
| 2,000 g | Natriumlaurylsulfat |
| 2,000 g | Isopropylalkohol |
| 0,160 g | (4-(Ethyl((2-hydroxyethyl)amino)-2-nitrophenyl)-amino)-ethanol-Hydrochlorid (HC Blue No. 12) |
| 0,170 g | 3-((2-Nitro-4-(trifluoromethyl)phenyl)amino)-1,2-propandiol (HC Yellow No. 6) |
| 0,012 g | 1-N-Hydroxyethylamino-4-methyl-2-nitrobenzol |
| 0,035 g | HC RED NO. 10 und HC RED NO. 11 (1:1) |
| ad 100,000 g | Wasser |

Die perlglänzende cremeartige Färbemasse wird mit Handschuhen auf das gewaschene und handtuchtrockene blonde Naturhaar aufgetragen. Nach einer Einwirkungszeit von 20 bis 25 Minuten wird die überschüssige Farbe mit Wasser und einem Shampoo herausgwaschen. Es wird ein schöner, glänzender mittelblonder Ton erreicht.

| **Beispiel 5:** | **nicht-oxidatives Haarfärbemittel** |
|---|---|
| 3,1 g | Cetylstearylakohol |
| 3,1 g | Stearylalkohol |
| 5,0 g | Kokosfettsäurediethanolamid (Cocamide DEA) |
| 2,0 g | Polyoxyethylen(30)oleylether (Oleth-30) |
| 0,5 g | Natriumlaurylsulfat |
| 7,0 g | Ethanol |
| 0,1 g | 1-N-Hydroxyethylamino-4-methyl-2-nitrobenzol |
| 0,5 g | HC RED NO. 10 und HC RED NO. 11 (1:1) |
| 0,2 g | 2-Amino-6-chlor-4-nitrophenol |
| ad 100,0 g | Wasser |

Die perlglänzende cremeartige Färbemasse wird mit Handschuhen auf das gewaschene und handtuchtrockene blonde Naturhaar aufgetragen. Nach einer Einwirkungszeit von 25 bis 30 Minuten wird die überschüssige Farbe mit Wasser und einem Shampoo herausgwaschen. Es wird ein glänzender, modischer Rotton erhalten.

Alle in der vorliegenden Anmeldung genannten Prozentangeaben stellen, soweit nicht anders angegeben, Gewichtsprozente dar.

## Patentansprüche

1. Farbträgermasse, enthaltend oxidative und/oder nicht-oxidative Farbstoffe, **dadurch gekennzeichnet, dass** sie eine Kombination aus
(a) 6,1 bis 20 Gewichtsprozent mindestens eines Fettalkoholes mit 14 bis 20 Kohlenstoffatomen,
(b) 6,1 bis 20 Gewichtsprozent mindestens eines Alkanolamides,
(c) 0,1 bis 15 Gewichtsprozent mindestens eines Fettalkoholalkoxylates oder Fettsäurealkoxylates und
(d) 0,1 bis 15 Gewichtsprozent mindestens eines anionischen Tensides enthält, wobei das Gewichtsverhältnis von Fettalkohol (a) zu Alkanolamid (b) gleich 0,5:2 bis 2:0,5 ist.

2. Farbträgermasse nach Anspruch 1, **dadurch gekennzeichnet, dass** der Fettalkohol (a) und das Alkanolamid (b) in einem Gewichtsverhältnis von 0,8:1,2 bis 1,2:0,8 vorliegen.

3. Farbträgermasse nach Anspruch 1 oder 2, **dadurch gekennzeichnet, dass** das Gewichtsverhältnis von Alkoxylat (c) zu anionischem Tensid (d) gleich 0,5:2 bis 2:0,5 ist:

4. Farbträgermasse nach einem der Ansprüche 1 bis 3, **dadurch gekennzeichnet, dass** der Fettalkohol (a) ausgewählt ist aus Cetylakohol, Stearylalkohol, Myristylalkohol, Isooctylalkohol und Isotridecylalkohol sowie Mischungen dieser Verbindungen.

5. Farbträgermasse nach einem der Ansprüche 1 bis 4, **dadurch gekennzeichnet, dass** das Alkanolamid (b) ausgewählt ist aus N-Acylderivaten des Monoethanolamins oder Diethanolamins.

6. Farbträgermasse nach einem der Ansprüche 1 bis 5, **dadurch gekennzeichnet, dass** das Alkoxylat (c) ausgewählt ist aus ethoxylierten Fettalkoholen oder Fettalkoholpolyglykoethern der Formel (I) CH3(CH2)x-O-(Ry)-H (I) [mit R= (CH2-CH2-O) oder (CH3-CH-CH2-O); x= C8 - C18 und y= 2 bis 300].

7. Farbträgermasse nach einem der Ansprüche 1 bis 6, **dadurch gekennzeichnet, dass** das anionisch Tensid ausgewählt ist aus Salzen und Estern von Carbonsäuren, Alkylethersulfaten, Alkylsulfaten, Fettalkoholethersulfaten, Sulfonsäuren und deren Salzen, Phosphorsäureestern und deren Salzen sowie Acylaminosäuren und deren Salzen.

8. Farbträgermasse nach einem der Ansprüche 1 bis 7, **dadurch gekennzeichnet, dass** der Fettalkohol (a), das Alkanolamid (b), das Alkoxylat (c) sowie das anionische Tensid (d) jeweils in einer Gesamtmenge von 7 bis 12 Gewichtsprozent enthalten ist.

9. Farbträgermasse nach einem der Ansprüche 1 bis 8, **dadurch gekennzeichnet, dass** sie frei von monomeren quaternären Ammoniumverbindungen sowie kationischen Emulgatoren undTensiden ist.

10. Farbträgermasse nach einem der Ansprüche 1 bis 9, **dadurch gekennzeichnet, dass** sie 0,1 bis 10 Gewichtsprozent Glykoldistearat enthält.

11. Mittel zur oxidativen Färbung von Haaren, **dadurch gekennzeichnet, dass** es durch Vermischen einer Farbträgermasse nach einem der Ansprüche 1 bis 10 mit einem Oxidationsmittel erhalten wird.

12. Mittel nach Anspruch 11, **dadurch gekennzeichnet, dass** das Oxidationsmittel Wasserstoffperoxid ist.

13. Verwendung einer Kombination aus
(a) 6,1 bis 20 Gewichtsprozent mindestens eines Fettalkoholes mit 14 bis 20 Kohlenstoffatomen,
(b) 6,1 bis 20 Gewichtsprozent mindestens eines Alkanolamides,
(c) 0,1 bis 15 Gewichtsprozent mindestens eines Fettalkoholalkoxylates oder Fettsäurealkoxylates und
(d) 0,1 bis 15 Gewichtsprozent mindestens eines anionischen Tensides enthält, wobei das Gewichtsverhältnis von Fettalkohol (a) zu Alkanolamid (b) gleich 0,5:2 bis 2:0,5 ist,
zur Erzeugung eines Perlglanzeffektes in Farbträgermassen und Färbemitteln für Keratinfasern.

## Claims

1. A dye carrier mass comprising oxidative and/or non-oxidative dyes, **characterized in that** it contains a combination of
a) 6.1 to 20% by weight of at least one fatty alcohol with 14 to 20 carbon atoms,
b) 6.1 to 20% by weight of at least one alkanolamide
c) 0.1 to 15% by weight of at least on fatty alcohol alkoxylate or fatty acid alkoxylate and
d) 0.1 to 15% by weight of at least one anionic surfactant
wherein the weight ratio of fatty alcohol (a) to alkanolamide (b) is 0.5:2 to 2:0.5.

2. The dye carrier mass according to Claim 1, **characterized in that** the fatty alcohol (a) and the alkanolamide (b) are present in a weight ratio of 0.8:1.2 to 1.2:0.8.

3. The dye carrier mass according to Claim 1 or 2, **characterized in that** the weight ratio of alkoxylate (c) to anionic surfactant (d) is 0.5:2 to 2:0.5.

4. The dye carrier mass according to any of Claims 1 to 3, **characterized in that** the fatty alcohol (a) is selected from cetyl alcohol, stearyl alcohol, myristyl alcohol, isooctyl alcohol, and isotridecyl alcohol as well as mixtures of these compounds.

5. The dye carrier mass according to any of Claims 1 to 4, **characterized in that** the alkanoamide (b) is selected from N-acyl derivatives of monoethanolamine or diethanolamine.

6. The dye carrier mass according to any of Claims 1 to 5, **characterized in that** the alkoxylate (c) is selected from ethoxylated fatty alcohols or fatty alcohol polyglyco ethers of the formula (I)
CH3(CH2)x-O-(Ry)-H (I)
[with R= (CH2-CH2-O) or (CH3-CH-CH2-O); x= C8-C18 and y= 2 through 300].

7. The dye carrier mass according to any of Claims 1 to 6, **characterized in that** the anionic surfactant is selected from salts and esters from carbonic acids, alkyl ether sulfates, alkyl sulfates and their salts, phosphoric acid esters and their salts, as well as acylamino acids and their salts.

8. The dye carrier mass according to any of Claims 1 to 7, **characterized in that** the fatty acid (a), the alkanolamide (b), the alkoxylate (c) as well as the anionic surfactant (d) is each contained in a total quantity of 7 to 12% by weight.

9. The dye carrier mass according to any of Claims 1 to 8, **characterized in that** it is free from monomer quaternary ammonium compounds as well as cationic emulsifiers and surfactants.

10. The dye carrier mass according to any of Claims 1 to 9, **characterized in that** it contains 0.1 to 10% by weight glycol distearate.

11. An agent for oxidative dying of hair, **characterized in that** it is obtained by mixing a dye carrier mass according to any of Claims 1 to 10 with an oxidizing agent.

12. The agent according to Claim 11, **characterized in that** the oxidizing agent is hydrogen peroxide.

13. Use of a combination containing
a) 6.1 to 20% by weight of at least one fatty alcohol with 14 to 20 carbon atoms,
b) 6.1 to 20% by weight of at least one alkanolamide
c) 0.1 to 15% by weight of at least on fatty alcohol alkoxylate or fatty acid alkoxylate and
d) 0.1 to 15% by weight of at least one anionic surfactant, wherein the weight ratio of fatty alcohol (a) to alkanolamide (b) is 0.5:2 to 2:0.5,
to create a pearlecent effect in dye carrier masses and dyes for keratin fibers.

## Revendications

1. Masse substrat de couleur, contenant des colorants oxydatifs et/ou non oxydatifs, **caractérisée en ce qu'**elle contient une combinaison de
(a) 6,1 à 20 % en poids d'au moins un alcool gras comportant 14 à 20 atomes de carbone,
(b) 6,1 à 20 % en poids d'au moins un alcanolamide,
(c) 0,1 à 15 % en poids d'au moins un alcoxylat d'alcool gras ou un alcoxylat d'acide gras et
(d) 0,1 à 15 % en poids d'au moins un tensioactif anionique, le rapport en poids de l'alcool gras (a) sur l'alcanolamide (b) étant égal à 0,5:2 à 2:0,5.

2. Masse substrat de couleur selon la revendication 1, **caractérisée en ce que** l'alcool gras (a) et l'alcanolamide (b) sont présents en un rapport en poids de 0,8:1,2 à 1,2:0,8.

3. Masse substrat de couleur selon la revendication 1 ou 2, **caractérisée en ce que** le rapport en poids de l'alcoxylat (c) sur le tensioactif anionique (d) est égal à 0,5:2 à 2:0,5.

4. Masse substrat de couleur selon l'une des revendications 1 à 3, **caractérisée en ce que** l'alcool gras (a) est choisi parmi l'alcool de cétyle, l'alcool de stéaryle, l'alcool de myristyle, l'alcool d'isooctyle et l'alcool d'isotridécyle et les mélanges de ceux-ci.

5. Masse substrat de couleur selon l'une des revendications 1 à 4, **caractérisée en ce que** l'alcanolamide (b) est choisi parmi les dérivés de N-acyle de mono-éthanolamine ou de diéthanolamine.

6. Masse substrat de couleur selon l'une des revendications 1 à 5, **caractérisée en ce que** l'alcoxylat (c) est choisi parmi les alcools gras éthoxylés ou les polyglyco-éthers d'alcools gras de formule (I)
CH3(CH2)x-O-(Ry)-H (I)
[dans laquelle R= (CH2-CH2-O) ou (CH3-CH-CH2-O) ; x = C8 à C18 et y = 2 à 300].

7. Masse substrat de couleur selon l'une des revendications 1 à 6, **caractérisée en ce que** le tensioactif anionique est choisi parmi les sels et esters d'acides carboxyliques, les sulfates d'alkyléther, les alkylsulfates, les sulfates d'éthers d'alcools gras, les acides sulfoniques et leurs sels, les esters d'acide phosphorique et leurs sels et les acides acylaminés et leurs sels.

8. Masse substrat de couleur selon l'une des revendications 1 à 7, **caractérisée en ce que** l'alcool gras (a), l'alcanolamide (b), l'alcoxylat (c) et le tensioactif anionique (d) sont contenus respectivement en une quantité totale de 7 à 12 % en poids.

9. Masse substrat de couleur selon l'une des revendications 1 à 8, **caractérisée en ce qu'**elle est dépourvue de composés d'ammonium quaternaire monomères et d'émulsifiants cationiques et de tensioactifs.

10. Masse substrat de couleur selon l'une des revendications 1 à 9, **caractérisée en ce qu'**elle contient 0,1 à 10 % en poids de distéarate de glycol.

11. Agent de coloration oxydative des cheveux, **caractérisé en ce qu'**il est obtenu par mélange d'une masse substrat de couleur selon l'une des revendications 1 à 10, avec un agent oxydant.

12. Agent selon la revendication 11, **caractérisé en ce que** l'agent oxydant est le peroxyde d'hydrogène.

13. Utilisation d'une combinaison de
(a) 6,1 à 20 % en poids d'au moins un alcool gras comportant 14 à 20 atomes de carbone,
(b) 6,1 à 20 % en poids d'au moins un alcanolamide,
(c) 0,1 à 15 % en poids d'au moins un alcoxylat d'alcool gras ou un alcoxylat d'acide gras et
(d) 0,1 à 15 % en poids d'au moins un tensioactif anionique, le rapport en poids de l'alcool gras (a) sur l'alcanolamide (b) étant égal à 0,5:2 à 2:0,5,
pour produire un effet nacré dans des masses substrats de couleur et agents colorants pour les fibres kératiniques.
